# EUROPEAN PATENT APPLICATION

(11) **EP 3 858 248 A1**
(43) Date of publication of application: **04.08.2021**
(21) Application number: 19870091.6
(22) Date of filing: 03.10.2019
(51) Int. Cl.: A61B 8/08

(54) **AUTOMATIC MEASURING METHOD FOR PERISTALTIC MOVEMENT, AUTOMATIC MEASURING PROGRAM FOR PERISTALTIC MOVEMENT, AUTOMATIC MEASURING DEVICE FOR PERISTALTIC MOVEMENT, AND AUTOMATIC MEASURING SYSTEM FOR PERISTALTIC MOVEMENT**

(30) Priority: 09.10.2018 JP 2018191304
(71) Applicant: Triple W Japan Inc., Tokyo 105-0001 (JP)
(72) Inventor: MASAMORI Ryosuke, Tokyo 105-0001 (JP); MURAKI Yosuke, Tokyo 105-0001 (JP)
(74) Representative: Puschmann Borchert Kaiser Klettner Patentanwälte Partnerschaft mbB
(86) International application number: PCT/JP2019/039174
(87) International publication number: WO 2020/075627

(57) **Abstract**

The present invention provides a device that allows a user to intuitively understand the activity of peristaltic movement of a gastrointestinal tract.

Provided is a method for automatic measurement of peristaltic movement using a computer. The method includes: an acquisition step S100 of acquiring measurement information about biological activity of one or more parts of a gastrointestinal tract; an extraction step S101 of extracting information about activity of the peristaltic movement from the measurement information about the biological activity acquired in the acquisition step S100; and a calculation step S102 of obtaining an activity score representing the degree of the activity of the peristaltic movement based on the information about the activity of the peristaltic movement.

## Description

### TECHNICAL FIELD

The present invention relates to a method, program, device, and system for automatic measurement of peristaltic movement of a gastrointestinal tract.

### BACKGROUND ART

Food taken in through the mouth passes through a gastrointestinal tract including organs such as the stomach, the small intestine, and the large intestine, and is eliminated out of the body as excrement. The gastrointestinal tract moves the food taken in (will be hereinafter referred to as "contents") by a phenomenon called peristalsis.

Patent Document 1 discloses an example of a technique of analyzing peristaltic movement.

Patent Document 1 discloses a peristaltic sound detection device. The device includes: a biological sound detector that detects biological sound made by the intestine; a frequency spectrum calculator that calculates a frequency spectrum of the biological sound; a matching coefficient calculator that matches the frequency spectrum of the biological sound with each of average frequency spectra of a plurality of peristaltic sounds to calculate a plurality of matching coefficients; and a peristaltic sound determiner that performs calculation processing of the plurality of matching coefficients to determine whether the biologic sound is the peristaltic sound or not. Patent Document 1 describes that this is a technique for discriminating the peristaltic sound from the biological sound.

### CITATION LIST

### PATENT DOCUMENT

PATENT DOCUMENT 1: Japanese Unexamined Patent Publication No. 2013-150723

### SUMMARY OF THE INVENTION

### TECHNICAL PROBLEM

According to the invention disclosed by Patent Document 1, expert knowledge is required for understanding the result of the analysis of the peristaltic movement. Thus, it is difficult for an ordinary person having no expert knowledge to intuitively understand the state of the peristaltic movement. However, it would be beneficial for the ordinary person if the person could accurately and intuitively understand the state of the peristaltic movement in everyday life, while taking relationship with excretion time into account, for example.

In view of the foregoing background, a primary object of the present invention is to accurately show the activity of the peristaltic movement in an intuitively perceptible manner.

### SOLUTION TO PROBLEM

In order to achieve the primary object, the present invention is directed to a method for automatic measurement of peristaltic movement using a computer. The method includes: an acquisition step of acquiring measurement information about biological activity of one or more parts of a gastrointestinal tract; an extraction step of extracting information about activity of the peristaltic movement from the measurement information about the biological activity acquired in the acquisition step; and a calculation step of obtaining an activity score representing the degree of the activity of the peristaltic movement based on the information about the activity of the peristaltic movement.

The activity score can represent the degree of the activity of the peristaltic movement in real time. The level of the activity score can be determined based on a threshold in the calculation step.

The method for automatic measurement of peristaltic movement according to the present invention can further include a display step of displaying at least the activity score on a screen of a computer terminal. A graph and/or a shape corresponding to the activity score can be displayed on the screen of the computer terminal in the display step. Further, for example, the activity score or the graph and/or the shape corresponding to the activity score can be displayed on the screen of the computer terminal together with an image of a human body including at least a region of the gastrointestinal tract in the display step.

Then, noise information contained in the measurement information acquired in the acquisition step can be removed to obtain the information about the activity of the peristaltic movement in the extraction step.

The method for automatic measurement of peristaltic movement can further include a notification step of giving notification to a user based on the activity score.

For example, ultrasonic waves can be transmitted into the body and reflected waves of the ultrasonic waves can be received to acquire the measurement information in the acquisition step.

The method for automatic measurement of peristaltic movement can further include a communication step of displaying the activity score or the graph and/or the shape corresponding to the activity score on a screen of a computer terminal of a user via a wired or wireless communication line.

The present invention further provides a computer program product with a built-in program for automatic measurement of peristaltic movement. The program is able to implement the method for automatic measurement of peristaltic movement after being loaded and executed by a computer.

The present invention further provides a device for automatic measurement of peristaltic movement. The device includes: an acquirer that acquires measurement information about biological activity of one or more parts of a gastrointestinal tract; and a calculator that extracts information about activity of the peristaltic movement from the information about the biological activity acquired by the acquirer, and obtains an activity score representing a degree of the activity of the peristaltic movement based on the information about the activity of the peristaltic movement.

The present invention further provides a system for automatic measurement of peristaltic movement implemented via a wired or wireless communication line. The system includes: an acquisition device that acquires measurement information about biological activity of one or more parts of a gastrointestinal tract; and a calculation device that extracts information about activity of the peristaltic movement from the information about the biological activity acquired by the acquisition device, and obtains an activity score representing a degree of the activity of the peristaltic movement based on the information about the activity of the peristaltic movement.

### ADVANTAGES OF INVENTION

Since the activity of the peristaltic movement of the gastrointestinal tract is shown to the user in an easily perceptible manner, the user can accurately and intuitively understand the activity of the peristaltic movement.

### BRIEF DESCRIPTION OF THE DRAWINGS

[FIG. 1] FIG. 1 is a flowchart of a method for automatic measurement of peristaltic movement according to an example of an embodiment of the present invention.
[FIG. 2] FIG. 2 is a general configuration of a device for automatic measurement of peristaltic movement according to an example of an embodiment of the present invention.
[FIG. 3] FIG. 3 is a flowchart of a calculator of the device for automatic measurement of peristaltic movement.
[FIG. 4] FIG. 4 is a flowchart of the calculator of the device for automatic measurement of peristaltic movement.
[FIG. 5] FIG. 5 is a flowchart of the calculator of the device for automatic measurement of peristaltic movement.
[FIG. 6] FIG. 6 shows an example of a chronological change of activity scores obtained by the device for automatic measurement of peristaltic movement.
[FIG. 7] FIG. 7 shows an example of a chronological change of activity scores obtained by the device for automatic measurement of peristaltic movement.
[FIG. 8] FIG. 8 shows an example of a chronological change of activity scores obtained by the device for automatic measurement of peristaltic movement.
[FIG. 9] FIG. 9 shows a screen of a display of the device for automatic measurement of peristaltic movement.

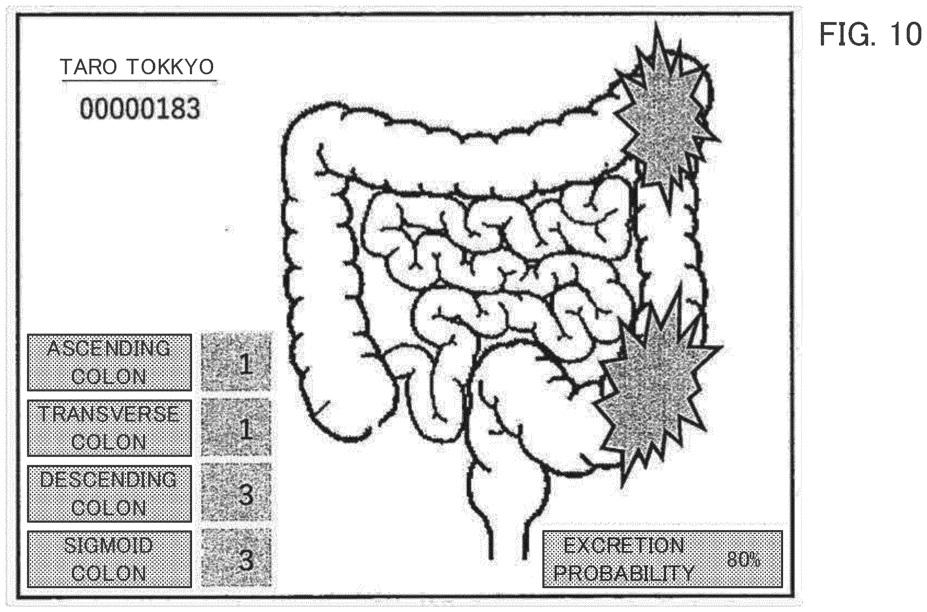

[FIG. 10] FIG. 10 shows a screen of the display of the device for automatic measurement of peristaltic movement.
[FIG. 11] FIG. 11 shows an example of information added to a shape corresponding to the activity score obtained by the device for automatic measurement of peristaltic movement.
[FIG. 12] FIG. 12 is a flowchart of the calculator of the device for automatic measurement of peristaltic movement.
[FIG. 13] FIG. 13 shows a screen of the display of the device for automatic measurement of peristaltic movement.
[FIG. 14] FIG. 14 shows a screen of the display of the device for automatic measurement of peristaltic movement.
[FIG. 15] FIG. 15 is a general configuration of a system for automatic measurement of peristaltic movement according to an example of an embodiment of the present invention.

### DESCRIPTION OF EMBODIMENTS

An embodiment of the present invention will be described below with reference to the attached drawings.

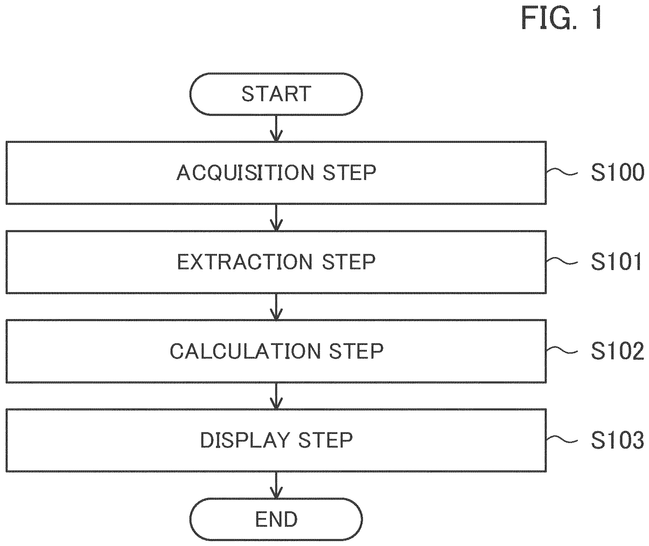

First, how a method for automatic measurement of peristaltic movement according to the present invention proceeds will be described with reference to FIG. 1. The method includes at least an acquisition step S100, an extraction step S101, and a calculation step S102.

### (1) Acquisition Step (S100);

The acquisition step S100 is a step of acquiring measurement information about biological activity of one or more parts of a gastrointestinal tract. In the acquisition step S100, for example, ultrasonic waves may be transmitted into the body and reflected waves of the ultrasonic waves may be received to obtain the measurement information.

### (2) Extraction Step (S101);

The extraction step S101 is a step of extracting information about the activity of the peristaltic movement from the measurement information about the biological activity acquired in the acquisition step S100. In the extraction step S101, for example, noise information contained in the measurement information acquired in the acquisition step S100 may be removed to obtain the information about the activity of the peristaltic movement.

### (3) Calculation step (S102);

The calculation step S102 is a step of obtaining an activity score representing the degree of the activity of the peristaltic movement based on the information about the activity of the peristaltic movement extracted in the extraction step S101. More specifically, in the calculation step S102, the level of the activity score is determined based on one or more thresholds. The activity score is information representing the degree of the activity of the peristaltic movement in real time. For example, although not particularly limited, the activity score may be a character, a symbol, or signal information. The term "real time" means the same point of time as the time of acquisition of the measurement information in the acquisition step S100. Alternatively, the term "real time" may include a predetermined period of time (e.g., several minutes) from the same point of time to a past point of time.

The method of the present invention may further include the following display step S103 in addition to the acquisition step S100, the extraction step S101, and the calculation step S102.

### (4) Display Step (S103);

The display step S103 is a step of displaying at least the activity score on a screen of a computer terminal. In the display step S103, a graph and/or a shape corresponding to the activity score may additionally be displayed on the screen of the computer terminal. Further, in the display step S103, the activity score or the graph and/or the shape corresponding to the activity score may be displayed on the screen of the computer terminal together with an image of a human body including at least a region of the gastrointestinal tract.

### (5) Notification Step (not shown);

The present invention may further include a notification step (not shown) of giving notification to a user based on the activity score. For example, when the degree of the activity of the peristaltic movement is high, the user may be notified of it.

### (6) Communication Step (not shown);

The present invention may further include a communication step (not shown) of displaying the activity score or a graph and/or a shape corresponding to the activity score via a wired or wireless communication line on a screen of a computer terminal of the user.

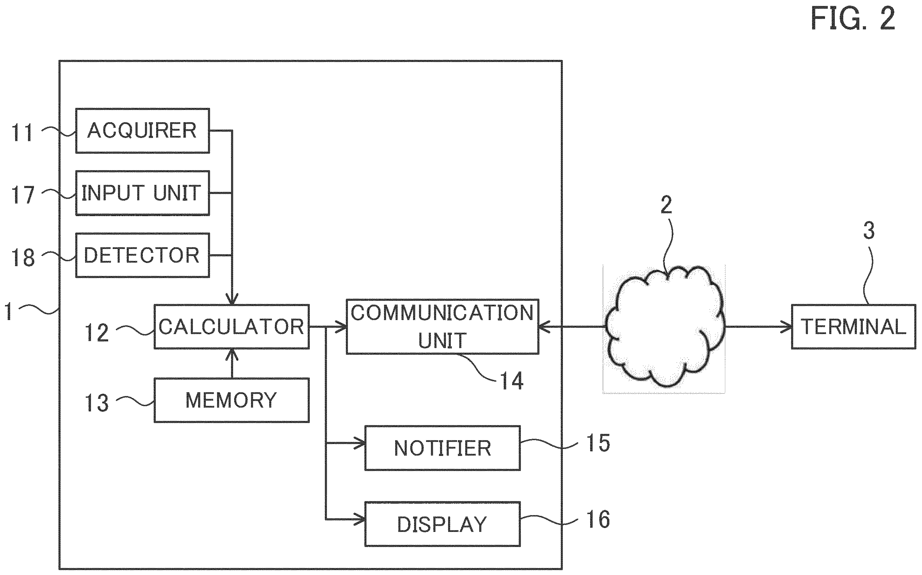

The method for automatic measurement of peristaltic movement according to the present invention can be implemented by a program, a device, or a system. FIG. 2 shows a general configuration of an example of an automatic peristalsis measurement device 1 usable in the present invention.

The automatic peristalsis measurement device 1 shown in FIG. 2 is a device using a computer, and includes an acquirer 11, a calculator 12, a memory 13, a communication unit 14, a notifier 15, a display 16, an input unit 17, and a detector 18. The acquirer 11 acquires measurement information about biological activity of the user. The calculator 12 extracts information about the activity of the peristaltic movement, and obtains an activity score representing the degree of the activity of the peristaltic movement. The notifier 15 gives notification to the user based on the calculation results. The display 16 shows at least the activity score. The memory 13 stores information required for the processing of the automatic peristalsis measurement device 1. The input unit 17 receives information entered by the user. The detector 18 detects information about the user's action. The communication unit 14 communicates with a terminal 3 via a wired or wireless communication line 2.

The automatic peristalsis measurement device 1 is not limited to a device exclusive for the measurement of the peristaltic movement. The automatic peristalsis measurement device 1 may use, for example, a server, a tablet, or a smartphone. For example, when the smartphone is used, a program that executes the processing shown in FIG. 1 may be installed in a storage of the smartphone. In this case, the display 16 may be a display of the smartphone.

### <Acquirer>

The acquirer 11 acquires the measurement information about the biological activity. The measurement information can be acquired by using, for example, an ultrasonic measurement technology. Examples of the ultrasonic measurement technology include continuous wave doppler (CWD). The continuous wave doppler is a technique of continuously transmitting and receiving ultrasonic waves, and analyzing the difference between the frequency of the transmitted ultrasonic waves and the frequency of the received ultrasonic waves. The larger movement the subject makes, the greater the difference is. Conversely, the smaller movement the subject makes, the smaller the difference is. The continuous wave doppler is used to measure the direction and velocity of a blood flow in the medical field. The acquirer 11 includes an ultrasonic wave transmitter (not shown) and an ultrasonic wave receiver (not shown). The ultrasonic wave transmitter transmits the ultrasonic waves into the body, and the ultrasonic wave receiver receives the reflected waves of the ultrasonic waves. The acquirer 11 analyzes the difference between the frequency of the ultrasonic waves transmitted by the ultrasonic wave transmitter and the frequency of the reflected waves received by the ultrasonic wave receiver in the acquisition step, thereby acquiring the measurement information about the biological activity. The greater the biological activity is, the greater the difference between the transmitted ultrasonic waves and the received reflected waves is. The smaller the biological activity is, the smaller the difference between the transmitted ultrasonic waves and the received reflected waves is.

The acquirer 11 may include, for example, a probe (not shown) that transmits and receives the ultrasonic waves. In this case, the probe that transmits and receives the ultrasonic waves can be arranged on the skin of the user's abdomen. An element of the probe transmits the ultrasonic waves toward the user's abdomen, and receives the reflected waves of the transmitted ultrasonic waves. Thus, the acquirer 11 acquires the measurement information about the biological activity of the abdomen in the acquisition step.

The acquirer 11 may be arranged on the skin as described above, or may be noncontact with the skin. The acquirer 11 is physically separable from the automatic peristalsis measurement device 1. The acquirer 11 may be inserted into the body of the user.

The acquirer 11 may be arranged at one part, or two or more acquirers 11 may be arranged at two or more parts.

The acquirers 11 are desirably arranged at parts suitable for achieving the purpose of acquisition. For example, in the case of predicting the probability of excretion of the contents, the measurement information is desirably acquired from four parts of the gastrointestinal tract, i.e., the ascending colon, the transverse colon, the descending colon, and the sigmoid colon. Combining the pieces of measurement information from the four parts is suitable for the prediction of the probability of excretion of the contents. Thus, the acquirers 11 of the automatic peristalsis measurement device 1 are arranged at positions corresponding to the ascending colon, transverse colon, descending colon, and sigmoid colon of the gastrointestinal tract. A method of predicting the probability of the excretion of the contents using the combination of the pieces of measurement information of the four parts will be described below.

### <Calculator>

The calculator 12 performs the extraction step of extracting information about the activity of the peristaltic movement from the measurement information about the biological activity acquired by the acquirer 11 in the acquisition step. The calculator 12 further performs a calculation step of obtaining the activity score based on the information about the activity of the peristaltic movement. For example, when the automatic peristalsis measurement device 1 is a smartphone or a server, a CPU or a memory corresponds to the calculator 12. Alternatively, a microcomputer or a field-programmable gate array (FPGA) can be the calculator 12.

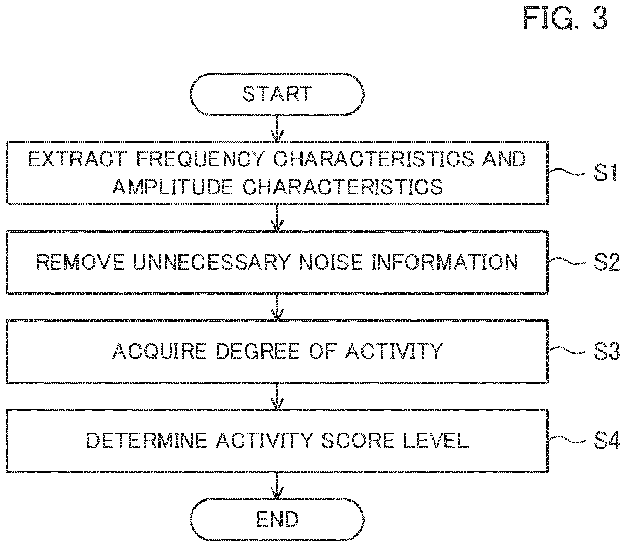

FIG. 3 is a flowchart of the extraction step (including steps S1 and S2) and the calculation step (including steps S3 and S4) performed by the calculator 12. First, in the extraction step, the calculator 12 extracts the information about the activity of the peristaltic movement from the measurement information acquired by the acquirer 11 in the acquisition step. More specifically, in the extraction step, the calculator 12 extracts frequency characteristics and amplitude characteristics from the measurement information about the biological activity by a specific analytical method (step S1). Examples of the analytical method include fast Fourier transform (FFT), empirical mode decomposition (EMD), and use of a filter (such as a band pass filter, a high pass filter, and a low pass filter).

Then, in the extraction step, noise information unnecessary for obtaining the activity score is removed from the frequency characteristics and the amplitude characteristics extracted in step S1, thereby extracting the frequency characteristics and the amplitude characteristics corresponding to the activity of the peristaltic movement (step S2). The unnecessary noise information includes pieces of information related to the user's action and those unrelated to the user's action. Examples of the pieces of information related to the user's action include frequency characteristics associated with the user's respiration, pulse, change in posture, walking, exercise, and turning over in bed, friction between clothes and the acquirer, and contact between the user and the acquirer. Examples of the noise information unrelated to the user's action include frequency characteristics associated with the movement of wind, and vibration of a vehicle that the user takes.

The detector 18 that detects the noise information related to the user's action (e.g., change in posture, walking, exercise, and turning over in bed) contained in the acquired measurement information may be provided. For example, an acceleration sensor or a gyro sensor can be used as the detector 18. Any known device may be used as the detector 18. Based on the noise information related to the user's action detected by the detector 18, the calculator 12 removes in the extraction step the noise information unnecessary for obtaining the activity score from the measurement information about the biological activity acquired by the acquirer 11. Thus, the calculator 12 can obtain the information about the activity of the peristaltic movement more accurately.

Subsequently, in the calculation step, the calculator 12 focuses on the frequency characteristics and the amplitude characteristics corresponding to the activity of the peristaltic movement and data of duration of the peristaltic movement, thereby acquiring the degree of activity of the peristaltic movement (step S3).

Then, in the calculation step, the calculator 12 acquires the activity score based on the information about the activity of the peristaltic movement (e.g., the frequency characteristics corresponding to the activity of the peristaltic movement). More specifically, the calculator 12 determines the level of the activity score based on one or more (e.g., five) thresholds (step S4). For example, when the frequency is higher than a predetermined threshold, the activity score can be determined to be high. When the amplitude is higher than a predetermined threshold, the activity score can be determined to be high. Further, the higher activity score indicates the more active peristaltic movement, and the lower activity score indicates the milder peristaltic movement.

The calculator 12 may make a determination of information about the contents in the gastrointestinal tract based on the obtained activity score. Examples of the information about the contents include whether the contents are present or not, the position of the contents, the moving speed of the contents, the probability of excretion of the contents (e.g., excretion probability when the user pushes down in a bathroom), and time until the contents are excreted.

The calculator 12 may determine whether the contents are present or not by analyzing the extracted information about the activity of the peristaltic movement. For example, if the contents are present at part of the gastrointestinal tract making the peristaltic movement, the information about the activity of the peristaltic movement is more likely to contain specific information, such as a high speed signal (high frequency component). This is because the contents in a liquid state are moving in the gastrointestinal tract. If no contents are present at part of the gastrointestinal tract making the peristaltic movement, the information about the activity of the peristaltic movement is less likely to contain specific information, such as a high speed signal (high frequency component).

The thresholds for determining the level of the activity score may be stored in the memory 13 which will be described later. The thresholds may be obtained by dividing a range of values of the information about the activity from the minimum to the maximum into equal subranges. Alternatively, a range of the threshold for a specific activity score may be set wider, and ranges of the thresholds for other activity scores may be set narrower. For example, if a minor change in the activity affects the determination of the activity score, the range of the threshold can be set narrower. Conversely, even if the activity greatly varies, the range of the threshold can be set wider as long as the variation does not affect the determination of the activity score.

The threshold greatly varies between individuals, and can be changed as needed. For example, some people would have the activity score of five at the maximum, and others three at the maximum. The threshold can be changed depending on such differences.

The automatic peristalsis measurement device 1 may include the input unit 17 to prompt the user to enter the information about the contents. Examples of the information entered by the user include information about food and drink taken in through the mouth, and information about the excretion. Examples of the information about the food and drink taken in through the mouth include time when the food and drink were taken in through the mouth, types of the food and drink (e.g., vegetables and meat), and the amount of the food and drink (e.g., the ratio of the food and drink the user taken to the whole amount of the food and drink served). Examples of the information about the excretion include time when the user excreted, time when the user felt a defecation desire, the amount of excrement (e.g., a metaphorical expression using the number of bananas), and hardness of the excrement (e.g., classification of stool properties according to the Bristol stool form scale).

Using these pieces of information as the input information of the calculator 12, it can be expected that the information about the contents is inferred with improved certainty. For example, time when the gastrointestinal tract makes the peristaltic movement can be predicted based on the time when the food and drink were taken in through the mouth. Since the moving speed of the contents varies depending on the type of the food and drink, the probability of the excretion of the contents can be predicted more accurately based on the type of the food and drink. The size of the contents can be estimated based on the amount of the food and drink. Since the harder excrement indicates that the longer digestion time are required, time required for the contents to move in the gastrointestinal tract can be estimated based on the hardness of the excrement. Further, time of the next excretion is can be predicted based on the time when the user excreted.

The activity score may be obtained from a single part or two or more parts of the gastrointestinal tract. In the following description, for example, the activity scores are obtained from four parts, namely, the ascending colon, the transverse colon, the descending colon, and the sigmoid colon.

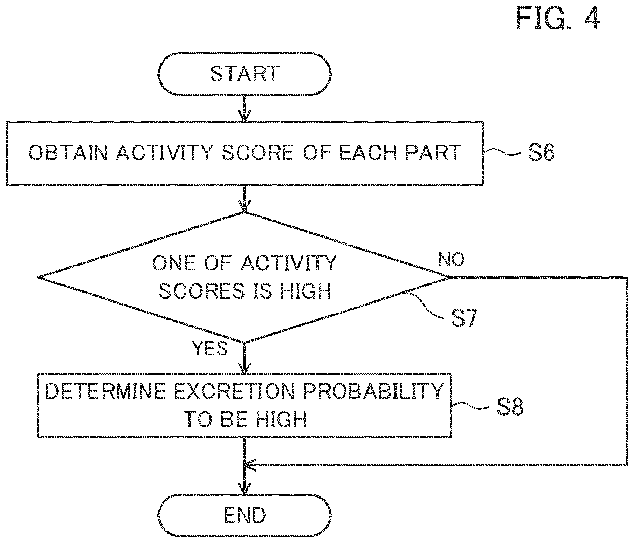

FIG. 4 shows the flow of processing of predicting the probability of excretion based on the activity scores of the four parts. First, the activity scores of the four parts are obtained (step S6). Then, if at least one of the four activity scores exceeds a certain value (YES is selected in step S7), the probability of the excretion of the contents is determined to be high (step S8).

In this example, the probability of the excretion is determined to be high if at least one of the four activity scores exceeds a certain value. For example, the excretion probability may be determined to be high if the activity score of the sigmoid colon close to the anus is high, or the probability of the excretion of the contents may be determined to be high if the activity score of the ascending colon far from the anus is high.

The part having the high activity score and the excretion probability may have a certain relationship. For example, some people excrete soon after the activity score of the part far from the anus has increased. On the other hand, some people do not excrete unless the activity score of the part near the anus increases. For this reason, the memory 13 which will be described later may store history information about the relationship between the part with the increased activity score and the excretion probability. This is expected to improve the certainty of the prediction of the excretion.

In the prediction of the excretion of the contents, a weighting factor may be set for each of the parts for which the activity of the peristaltic movement is calculated. For example, the weighting factor for the sigmoid colon near the anus and that for the ascending colon far from the anus may be different values. This is expected to improve the certainty of the prediction of the excretion. For example, suppose that both of the ascending colon far from the anus and the sigmoid colon near the anus have the activity score of three. In general, the latter shows the higher probability of excretion of the contents because the sigmoid colon is closer to the anus. Thus, even if the ascending colon and the sigmoid colon have the same activity scores, the probability of the excretion differs. Therefore, in this case, different weighting factors may be set for the ascending colon and the sigmoid colon. Such addition of the weighting factors lowers the activity score of the ascending colon far from the anus, and raises the activity score of the sigmoid colon near the anus.

The probability of the excretion of the contents may be predicted based on a total activity score. The total activity score is, for example, the sum of the activity scores of the different parts of the gastrointestinal tract. If the ascending colon has the activity score of three, the transverse colon has two, the descending colon has one, and the sigmoid colon has one, the total activity score is seven (3 + 2 + 1 + 1 = 7).

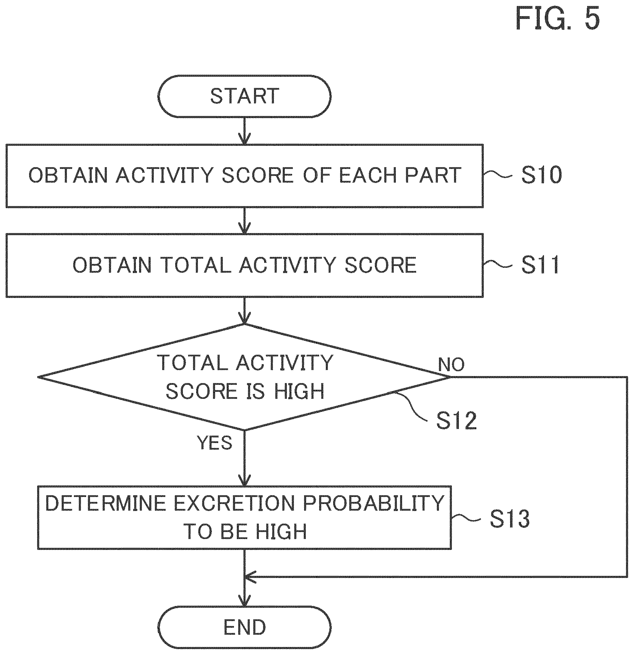

FIG. 5 shows the flow of processing of predicting the excretion by the calculator 12 based on the total activity score. First, the activity scores are obtained from the parts of the gastrointestinal tract (step S10). Then, the activity scores are added up to obtain the total activity score (step S11). Then, if the total activity score exceeds a predetermined threshold (YES is selected in step S12), the probability of the excretion of the contents is determined to be high (step S13).

The results of the determination shown in FIGS. 4 and 5 can be used to give the user an alert of a first grade (first alert), which is one of alerts of different grades to be given to the user. The first alert is given to the user based on the determination result of any one of the activity scores shown in FIG. 4, and then a final alert having higher certainty than the first alert can be given to the user based on the determination result of the other activity score. Alternatively, the first alert may be given to the user based on the determination result of the total activity score shown in FIG. 5 in addition to one of the activity scores shown in FIG. 4.

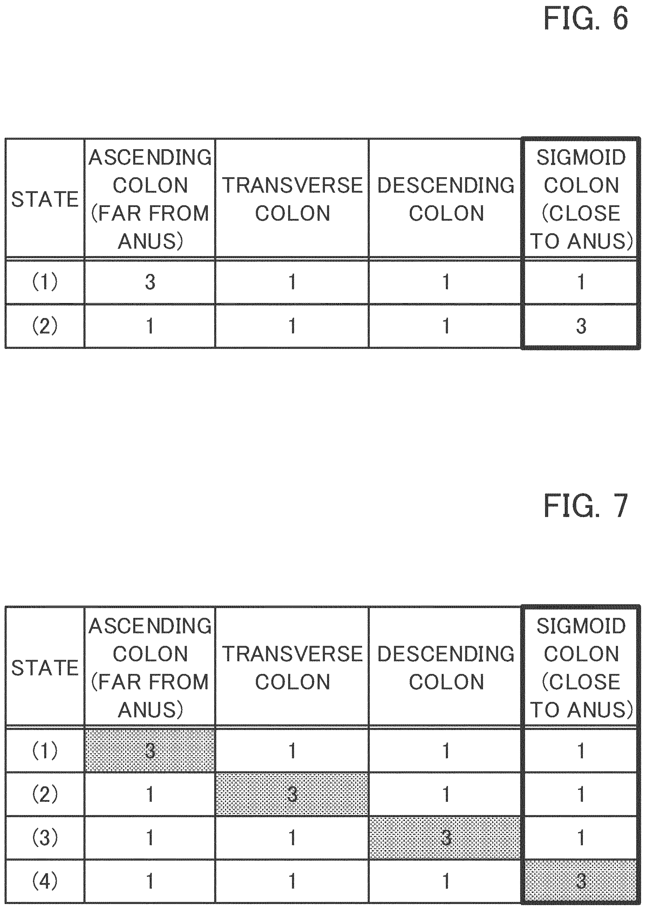

The probability of the excretion of the contents may be predicted based on a chronological change in the activity score. For example, as shown in FIG. 6, if "the ascending colon, the transverse colon, the descending colon, and the sigmoid colon" arranged in descending order of distance from the anus have the activity scores that change from the state (1) "3, 1, 1, 1" to the state (2) "1, 1, 1, 3," the calculator 12 predicts that the contents will be excreted with high probability. This is because the contents can be inferred to be present near the anus from the increase in the activity score of the sigmoid colon near the anus.

The peristaltic movement of the gastrointestinal tract causes the contents to move to the rectum in principle. Thus, the contents are more likely to move to the rectum. However, the contents do not necessarily move to the rectum. The contents go toward the rectum while repeatedly moving up and down due to the peristaltic movement of the gastrointestinal tract. Thus, the contents may sometimes move in the direction opposite to the rectum. If the peristaltic movement is too active or irregular, the gastrointestinal tract cannot cause the contents to move smoothly.

Thus, the calculator 12 may infer the position or moving direction of the contents based on the chronological change in the activity score. For example, as shown in FIG. 7, suppose that "the ascending colon, the transverse colon, the descending colon, and the sigmoid colon" arranged in descending order of distance from the anus have the activity scores that change in the order of the states (1), (2), (3), and (4), i.e., "3, 1, 1, 1" in the state (1), "1, 3, 1, 1" in the state (2), "1, 1, 3, 1" in the state (3), and "1, 1, 1, 3" in the state (4).

Referring to FIG. 7, in the state (1), the ascending colon far from the anus has the high activity score of three, and the sigmoid colon near the anus has the low activity score of one. Since the contents are inferred to be present at the part with the high activity score, the contents are inferred to be present at the part far from the anus. Thereafter, as the state changes in the order of (2), (3), and (4), the part with the high activity score shifts closer to the anus. It can be inferred from this phenomenon that the contents are moving to the anus.

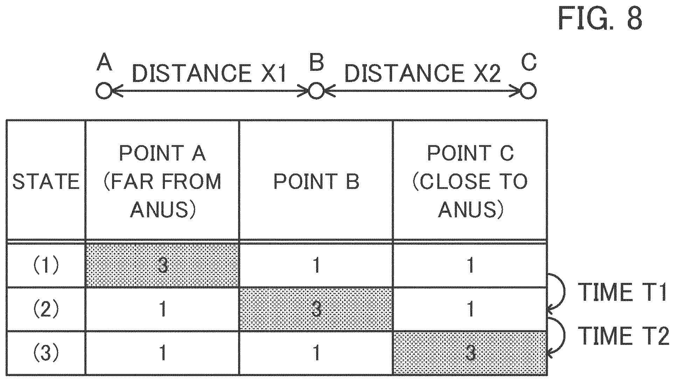

Further, the calculator 12 may predict the probability of the excretion of the contents based on the inferred distance traveled by the contents, time required for the travel, and the length of the gastrointestinal tract. As shown in FIG. 8, suppose that the activity scores are obtained from three parts, i.e., points A, B, and C in descending order of distance from the anus, and the contents are excreted when moved to the point C. The distance between the points A and B is regarded as X1, and the distance between the points B and C as X2.

Referring to FIG. 8, suppose that the activity scores of these points vary in the order of the following states (1), (2), and (3). For example, the activity scores are "3, 1, 1" in the state (1), "1, 3, 1" in the state (2), and "1, 1, 3" in the state (3). Suppose that time taken by the change from the state (1) to the state (2) is T1, and time taken by the change from the state (2) to the state (3) is T2. Since the contents can be inferred to be present at the part with the high activity score, the contents can be inferred to move from the point A to the point B, and then from the point B to the point C. In other words, the contents take time T1 to move from the point A to the point B, and take time T2 to move from the point B to the point C.

The moving speed S at which the contents move from the point A to the point B can be calculated by dividing the distance X1 between the points A and B by the time T1 required for the contents to move from the point A to the point B. Suppose that the contents move at the same speed between the points A and B and between the points B and C, the time T2 required for the contents to move from the point B to the point C can be calculated by dividing the distance X2 between the points B and C by the moving speed S.

In the above-described example, the contents are supposed to move at the same speed between the points A and B and between the points B and C. However, even if the contents move at different speeds, the time T2 required for the contents to move from the point B to the point C can be calculated. For this purpose, the memory 13 which will be described later may store history information about the moving speed. A future moving speed can be predicted by referring to the history information of the moving speed. For example, the time T2 required for the contents to move from the point B to the point C can be calculated by dividing the distance X2 between the points B and C by a moving speed S2 at which the contents move between the points B and C.

### <Display>

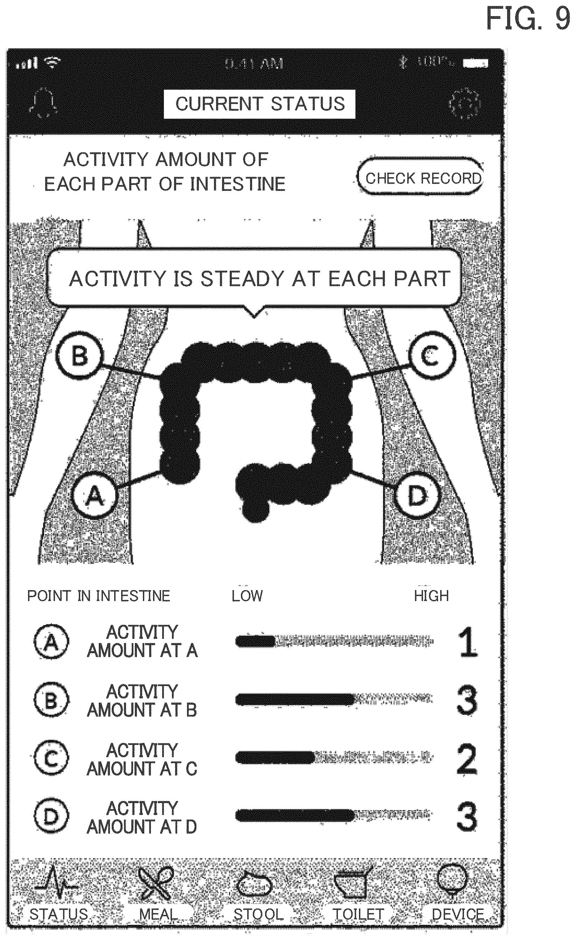

The display 16 performs a display step of displaying at least the activity score on a screen of a computer terminal as shown in FIG. 9. Further, the display 16 displays a graph and/or a shape corresponding to the activity score on the screen of the computer terminal in the display step. In addition, in the display step, the display 16 displays the activity score or a graph and/or a shape corresponding to the activity score on the screen of the computer terminal together with an image of a human body including at least a region of the gastrointestinal tract. The display 16 does not necessarily show a model of the gastrointestinal tract as long as the region of the gastrointestinal tract is shown. The image of the human body may include a picture, a photograph, and a schematic diagram. This is advantageous because the user can intuitively understand the state of the peristaltic movement. When a smartphone is used as the automatic peristalsis measurement device 1, for example, its display screen corresponds to the display 16.

In the example of FIG. 9, an image of a human body including the region of the gastrointestinal tract (the large intestine) is shown on the center of the display 16. Four parts of the large intestine are indicated to show the activity scores (amount of activity) of the four parts (A, B, C, and D). The activity scores of the four parts are shown on the lower right of the image of the human body. A graph corresponding to the activity scores is shown on the left of the activity scores. Bars in the graph get longer with the increase in the activity scores. Thus, the user can intuitively understand the activity of the peristaltic movement. If one of the parts show a particularly high activity score, a message informing the user of it may be shown on the display 16.

In the example of FIG. 9, some icons indicating a meal, excrement, and a toilet are shown below the graph corresponding to the activity scores. When the user selects one of the icons, a dialog box that prompts the user to enter information associated with the selected icon may be displayed. For example, when the user selects the meal icon, a dialog box that prompts the user to enter information about food and drink that the user has taken in through the mouth can be displayed. When the user selects the excrement icon, a dialog box that prompts the user to enter information about the amount of excrement can be displayed. When the user selects the toilet icon, a dialog box that prompts the user to enter time when the user went to the bathroom can be displayed.

Alternatively, as shown in FIG, 10, the display 16 may show in the display step a shape corresponding to the activity score to overlap with the image of the human body, e.g., a schematic diagram of the gastrointestinal tract.

The shape corresponding to the activity score may be added with information such as color, a character, an alphanumeric number, a symbol, and an image. The shape may be moved like video, or colorless and transparent. Alternatively, the shape may be replaced with, for example, information such as color, a character, an alphanumeric number, a symbol, and an image.

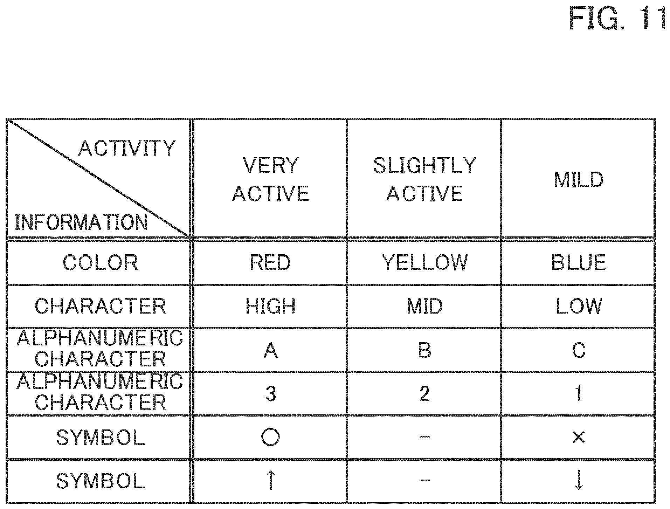

As an example of the color, as shown in FIG. 11, the shape may be colored in red when the activity score is high. Conversely, the shape may be colored in blue when the activity score is low. When the shape is overlapped with the model of the gastrointestinal tract, part of the gastrointestinal tract actively making the peristaltic movement can be turned red in the model of the gastrointestinal tract.

As an example of the character, a character that means "high" may be added to the shape when the activity score is high. Conversely, a character that means "low" may be added to the shape when the activity score is low.

As an example of the alphanumeric number, an alphanumeric number "A" or "3" may be added when the activity score is high. Conversely, an alphanumeric number "C" or "1" may be added when the activity score is low.

As an example of the symbol, a circle symbol or an upward arrow symbol may be added when the activity score is high. Conversely, an X symbol or a downward arrow symbol may be added when the activity score is low.

As an example of the image, an image of a widened gastrointestinal tract may be added when the activity score is high. Conversely, an image of a narrowed gastrointestinal tract may be added when the activity score is low.

As an image of the video, the gastrointestinal tract may be animated to have its width changing with time using an animation technology such as Graphics Interchange Format (GIF). The change in width may be shown larger in the part with the high activity score, and may be shown smaller in the part with the low activity score.

The shape itself may be changed. For example, the shape may be changed to a zigzag, or a crying icon when the activity score is high. Conversely, the shape may be changed to a sun icon, or a smiley icon when the activity score is low.

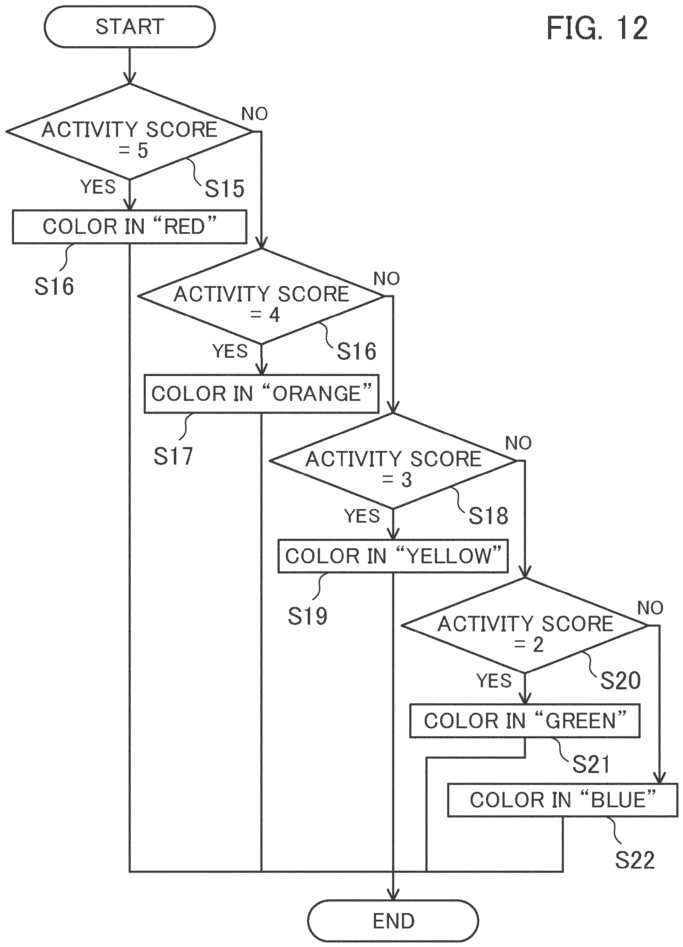

FIG. 12 shows an example of a flowchart of how the information to be added to the shape corresponding to the activity score is determined. In this example, the activity score has five grades, based on which the shape is colored. First, if the activity score is five (YES is selected in step S15), the shape is determined to be colored in red (step S16). If the activity score is not five (NO is selected in step S15), but four (YES is selected in step S16), the shape is determined to be colored in orange (step S17). If the activity score is not four (NO is selected in step S16), but three (YES is selected in step S18), the shape is determined to be colored in yellow (step S19). If the activity score is not three (NO is selected in step S18), but two (YES is selected in step S20), the shape is determined to be colored in green (step S21). If the activity score is not two (NO is selected in step S20), it is one, and the shape is determined to be colored in blue (step S22).

In the display step, the display 16 may add a combination of various pieces of information to the shape. For example, a number "3" may be added to the shape and shown in red when the activity score is high.

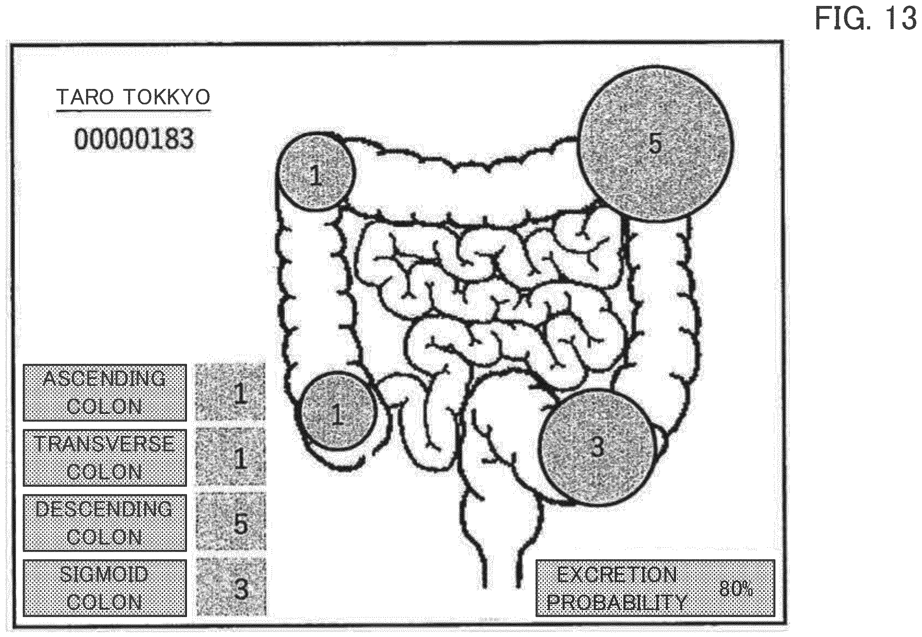

Further, in the display step, the display 16 may change an area of the shape corresponding to the activity score based on the activity score. As shown in FIG. 13, the area of the shape can be shown larger with the increase in the activity score. Conversely, the area of the shape can be shown smaller with the decrease in the activity score.

The difference in size of an object having a particular shape can be recognized more intuitively than the difference in number or character the meaning of which people need to understand. Thus, when the display 16 changes the area of the displayed shape corresponding to the activity score in the display step, the user can intuitively understand the degree of the activity, which is advantageous.

In the display step, the display 16 can show not only the shape corresponding to the activity score, but also a shape corresponding to the contents. For example, a shape added with color, a character, an alphanumeric character, a symbol, or an image can be shown at the position of the contents.

For example, if the contents are inferred to be present in the descending colon, the shape of the contents may be shown at the position of the descending colon in the image of the human body. Alternatively, a number indicating the size of the contents may be shown in place of the shape at the position where the contents are inferred to be present. If there are several pieces of contents in the gastrointestinal tract, alphabets "A," "B," and "C," numbers "1," "2," and "3," or circle and x symbols may be shown to distinguish these contents.

Further, as shown in FIG, 10, the display 16 may show in the display step a number representing the activity score or the excretion probability.

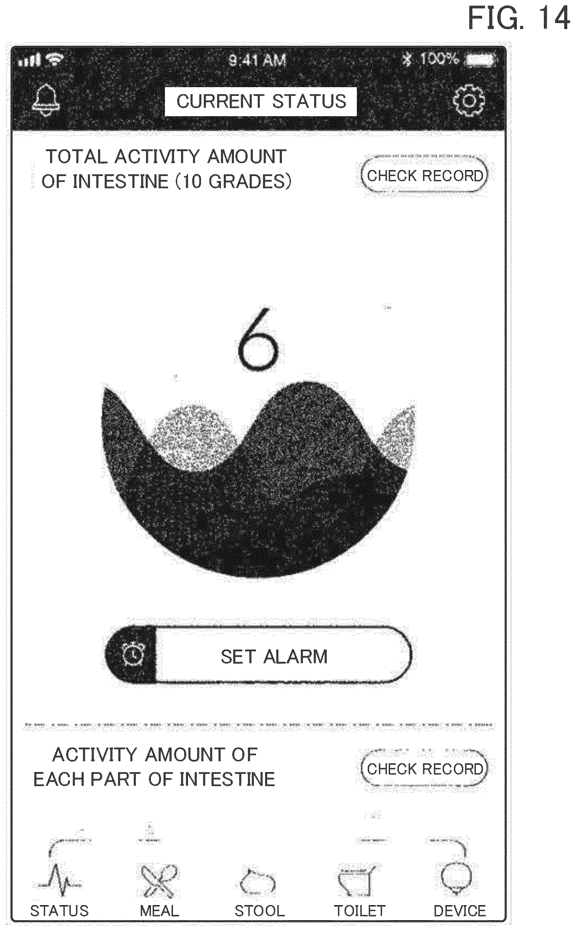

FIG. 14 shows an example of the total activity score (total amount of activity) shown by the display 16 in the display step. The total activity score in this example shown in FIG. 14 is irrelevant to that shown in FIG. 9.

### <Communication Unit>

The communication unit 14 performs a communication step of displaying the activity score or a graph and/or a shape corresponding to the activity score via a wired or wireless communication line on a screen of a computer terminal 3 of the user. When a smartphone is used as the automatic peristalsis measurement device 1, a wireless LAN adapter or a Bluetooth (registered trademark) adapter, for example, can be the communication unit 14.

Examples of the terminal 3 that communicates with the communication unit 14 include a personal computer, a tablet, and a smartphone. The activity score determined by the calculator 12 may be shown on the screen of the terminal 3 instead of the display 16. The terminal 3 may show an image of a human body, or may give an alert about the excretion.

Alternatively, the user may be prompted to enter the information that should be entered to the input unit 17 to the terminal 3. For example, the user may be prompted to enter information, such as time when the user took in food and drink through the mouth, types of the food and drink (e.g., vegetables and meat), and time when the user excreted, to the terminal 3.

### <Notifier>

The notifier 15 performs a notification step of giving notification to the user based on the activity score. When a smartphone is used as the automatic peristalsis measurement device 1, a speaker, for example, can be the notifier 15.

For example, when the activity of the peristaltic movement is high, the user is notified of it. When the peristaltic movement is too active or irregular, for example, the user is notified of it. When the colon is in a constipated state, for example, the user is notified of it. When the probability of excretion of the contents is predicted to be high, for example, the user is notified of it.

The notification step is implemented by, for example, light, sound, or vibration. For example, the light may be made by turning an LED light on, the sound may be made by sounding a buzzer, and the vibration may be made by turning a vibrator on.

### <Memory>

The memory 13 stores information required for the processing of the automatic peristalsis measurement device 1. When a smartphone is used as the automatic peristalsis measurement device 1, a storage, for example, can be the memory 13.

Examples of the information about the acquirer 11 stored in the memory 13 include the measurement information (e.g., frequency characteristics, amplitude characteristics, voltage, or an amplification factor), and the number or position of parts to be measured.

Examples of the information about the calculator 12 stored in the memory 13 include information about the peristaltic movement, information about the contents, and information about the gastrointestinal tract. Examples of the information about the peristaltic movement include the threshold of the activity score, the history of the activity score, time during which the peristaltic movement was made, the weighting factor for each part to be measured, and a pattern of a single activity score or patterns of combinations of a plurality of activity scores. Examples of the information about the contents include whether the contents are present or not, the position of the contents, types of the contents (meat and vegetables), and the moving speed of the contents. Examples of the information about the gastrointestinal tract include the length of the gastrointestinal tract.

Examples of the information about the communication unit 14 stored in the memory 13 include information for identifying a terminal to communicate with (e.g., IP address), and information to communicate.

Examples of the information about the notifier 15 stored in the memory 13 include information about the notification, information about the notification by light, information about the notification by sound, and information about the notification by vibration. Examples of the information about the notification include time when notification will be or was made, and types of notification (e.g., a symptom alert or a final alert). Examples of the information about the notification by light include color of the light, and a blinking time interval of the light. Examples of the information about the notification by sound include a pitch of the sound, and time during which the sound is made. Examples of the notification by vibration include the magnitude of the vibration and a vibrating time interval.

Examples of the information about the display 16 stored in the memory 13 include a graph and/or a shape corresponding to the activity score, and an image of a human body including at least the region of the gastrointestinal tract.

Examples of the information about the input unit 17 stored in the memory 13 include information about food and drink taken in through the mouth, and information about excretion.

An embodiment of the present invention can be implemented as a computer program product that is accessible from a medium usable or readable by a computer. The computer program may be stored in a recording medium such as a CD-ROM, or can be downloaded to the terminal via the Internet. For example, when the user uses a smartphone to implement the present invention, the user may download the computer program product to the smartphone via the Internet, and install and execute an automatic peristalsis measurement program in the computer program product, thereby implementing the present invention.

An embodiment of the present invention is a recording medium that stores the automatic peristalsis measurement program. The program stored in the recording medium is read by the CPU, and the same processing as that described above is executed under the control by the CPU.

The program can be stored using various types of non-transitory computer readable media and supplied to the computer. The non-transitory computer readable media include various types of tangible storage media. Examples of the non-transitory computer readable media include a magnetic recording medium (e.g., a flexible disk, a magnetic tape, and a hard disk), a magneto-optical recording medium (e.g., a magneto-optical disk), a Compact Disc Read Only Memory (CD-ROM), CD-R, CD-R/W, and a semiconductor memory (e.g., a mask ROM, a Programmable ROM (PROM), an Erasable PROM (EPROM), a Flash ROM, and a Random Access Memory (RAM)). Further, the program may be supplied to the computer by various types of transitory computer readable media. Examples of the transitory computer readable media include an electric signal, an optical signal, and electromagnetic waves. The transitory computer readable medium can supply the program to the computer via a wired communication path such as an electric wire and an optical fiber or a wireless communication path.

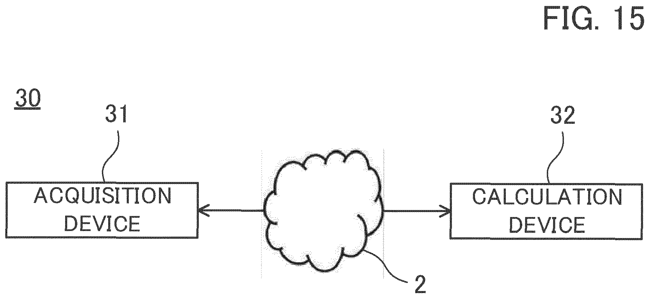

An embodiment of the present invention may be a system implemented via a wired or wireless communication line. FIG. 15 shows a general configuration of a system for automatic measurement of peristaltic movement according to an example of the embodiment of the present invention.

As shown in FIG. 15, an automatic peristalsis measurement system 30 includes an acquisition device 31 and a calculation device 32. The acquisition device 31 and the calculation device 32 are connected together via the wired or wireless communication line 2. The acquisition device 31 acquires, in an acquisition step, measurement information about biological activity of one or more parts of the gastrointestinal tract in the user's body.

The calculation device 32 extracts, in an extraction step, information about the activity of the peristaltic movement from the measurement information about the biological activity acquired by the acquisition device 31. The calculation device 32 obtains in a calculation step an activity score representing the degree of the activity of the peristaltic movement based on the information about the activity of the peristaltic movement. The steps performed by the acquisition device 31 and the calculation device 32 are the same as those performed by the acquirer 11 and calculator 12 of the automatic peristalsis measurement device 1 described above. The calculation device 32 can be implemented as, for example, a physical server or a virtual server. The automatic peristalsis measurement system 30 may include a plurality of acquisition devices 31 or a plurality of calculation devices 32.

Data acquired by the acquisition device 31 in the acquisition step may be transmitted to the calculation device 32 which is a computer in a cloud environment via a communication network. Further, data processed in the extraction step and the calculation step by the calculation device 32 which is a computer in the cloud environment may be transmitted to the user's computer terminal via the communication network. Then, the user's computer terminal may display the data on its screen in the display step.

The embodiments described above are premised on the measurement of the peristaltic movement of the large intestine. However, the gastrointestinal tract to be measured in the present invention is not limited to the large intestine. The present invention can be used for the measurement of the peristaltic movement of the gastrointestinal tract in the abdomen such as the stomach, the small intestine, and the large intestine.

One of the advantages of the present invention is the prediction of the probability of the excretion in everyday life. The present invention can be applied to patients having excretion disorders, elderly people having difficulty in going to the bathroom by themselves, or those having excretion problems not as serious as going to the hospital but having a poor quality of life (QOL). Grasping the probability of excretion, nurses, caregivers, certified care workers, and care managers can properly support the excretion.

As another advantage of the present invention, drugs such as laxatives can be suitably selected. There are various types of laxatives, for example, one that makes the peristaltic movement more active, one that increases the moisture in the body, one that swells the rectum, and one that stimulates the small intestine. A proper drug needs to be selected to relieve constipation. However, in actuality, the drugs are not properly selected in nursing homes because the peristaltic movement cannot be measured easily.

As another advantage of the present invention, the effect of the drug such as the laxative can be checked. For example, to a person having poor activity of the peristaltic movement, i.e., a symptom of constipation, a nurse gives a large intestine irritating laxative and can check its effect. The large intestine irritating laxative causes the peristaltic movement of the large intestine to promote defecation. If it is confirmed by the present invention that the administration of the large intestine irritating laxative has raised the activity of the peristaltic movement, the nurse can determine that the large intestine irritating laxative has worked. Thus, the nurse can suitably lead the patient to the bathroom.

As another advantage of the present invention, the effect of rehabilitation can be checked. The therapeutic effect of kinesitherapy or dietetic therapy can be easily checked.

As another advantage of the present invention, the effect of a drug in gastroscopy can be checked. The gastroscopy requires reduction of excessive peristaltic movement of the stomach for improved inspection efficiency. According to the present invention, whether the effect of the drug is sufficient or not can be checked through the measurement of the peristaltic movement of the stomach.

As another advantage of the present invention, whether progesterone is normally secreted or not during pregnancy can be checked. Progesterone secreted during pregnancy has been known to reduce the peristaltic movement of the small intestine. According to the present invention, whether progesterone is normally secreted or not can be checked through the measurement of the peristaltic movement of the small intestine.

The advantages described so far are merely examples and non-limitative, and the present invention may have other advantages.

The disclosed embodiments should be considered to be exemplary in all respects and non-limitative. The scope of the present invention is not defined by the above description, but the scope of the appended claims. Any modifications falling within the range of equivalents to the claims are all encompassed within the scope of the present invention.

The present invention can be modified in the following manner.
[1] A method for automatic measurement of peristaltic movement using a computer, the method including: an acquisition step of acquiring measurement information about biological activity of one or more parts of a gastrointestinal tract; an extraction step of extracting information about activity of the peristaltic movement from the measurement information about the biological activity acquired in the acquisition step; and a calculation step of obtaining an activity score representing a degree of the activity of the peristaltic movement based on the information about the activity of the peristaltic movement.
[2] The method of [1], wherein the activity score represents the degree of the activity of the peristaltic movement in real time.
[3] The method of [1] or [2], wherein a level of the activity score is determined based on a threshold in the calculation step.
[4] The method of any one of [1] to [3], further including a display step, wherein at least the activity score is displayed on a screen of a computer terminal in the display step.
[5] The method of [4], wherein a graph and/or a shape corresponding to the activity score is displayed on the screen of the computer terminal in the display step.
[6] The method of [4] or [5], wherein the activity score or a graph and/or a shape corresponding to the activity score is displayed together with an image of a human body including at least a region of the gastrointestinal tract on the screen of the computer terminal in the display step.
[7] The method of any one of [1] to [6], wherein noise information contained in the measurement information acquired in the acquisition step is removed to obtain the information about the activity of the peristaltic movement in the extraction step.
[8] The method of any one of [1] to [7], further including a notification step, wherein notification is given to a user based on the activity score in the notification step.
[9] The method of any one of [1] to [8], wherein ultrasonic waves are transmitted into the body and reflected waves of the ultrasonic waves are received to acquire the measurement information in the acquisition step.
[10] The method of any one of [1] to [9], further including a communication step, wherein the activity score or a graph and/or a shape corresponding to the activity score is displayed on a screen of a computer terminal of a user via a wired or wireless communication line in the communication step.
[11] A computer program product with a built-in program for automatic measurement of peristaltic movement, the program being able to implement the method of any one of [1] to [10] after being loaded and executed by a computer.
[12] A device for automatic measurement of peristaltic movement, the device including: an acquirer that acquires measurement information about biological activity of one or more parts of a gastrointestinal tract; and a calculator that extracts information about activity of the peristaltic movement from the information about the biological activity acquired by the acquirer, and obtains an activity score representing a degree of the activity of the peristaltic movement based on the information about the activity of the peristaltic movement.
[13] A system for automatic measurement of peristaltic movement implemented via a wired or wireless communication line, the system including: an acquisition device that acquires measurement information about biological activity of one or more parts of a gastrointestinal tract; and a calculation device that extracts information about activity of the peristaltic movement from the information about the biological activity acquired by the acquisition device, and obtains an activity score representing a degree of the activity of the peristaltic movement based on the information about the activity of the peristaltic movement.

### DESCRIPTION OF REFERENCE CHARACTERS

S100 Acquisition Step
S101 Extraction Step
S102 Calculation step
S103 Display Step
1 Automatic Peristalsis Measurement Device
11 Acquirer
12 Calculator
13 Memory
14 Communication Unit
15 Notifier
16 Display
17 Input Unit
18 Detector
30 Automatic Peristalsis Measurement System
31 Acquisition Device
32 Calculation Device
2 Communication Line
3 Terminal

## Claims

1. A method for automatic measurement of peristaltic movement using a computer, the method including:
an acquisition step of acquiring measurement information about biological activity of one or more parts of a gastrointestinal tract;
an extraction step of extracting information about activity of the peristaltic movement from the measurement information about the biological activity acquired in the acquisition step; and
a calculation step of obtaining an activity score representing a degree of the activity of the peristaltic movement based on the information about the activity of the peristaltic movement.

2. The method of claim 1, wherein the activity score represents the degree of the activity of the peristaltic movement in real time.

3. The method of claim 1 or 2, wherein a level of the activity score is determined based on a threshold in the calculation step.

4. The method of any one of claims 1 to 3, further including a display step, wherein
at least the activity score is displayed on a screen of a computer terminal in the display step.

5. The method of claim 4, wherein a graph and/or a shape corresponding to the activity score is displayed on the screen of the computer terminal in the display step.

6. The method of claim 4 or 5, wherein the activity score or a graph and/or a shape corresponding to the activity score is displayed together with an image of a human body including at least a region of the gastrointestinal tract on the screen of the computer terminal in the display step.

7. The method of any one of claims 1 to 6, wherein noise information contained in the measurement information acquired in the acquisition step is removed to obtain the information about the activity of the peristaltic movement in the extraction step.

8. The method of any one of claims 1 to 7, further including a notification step, wherein notification is given to a user based on the activity score in the notification step.

9. The method of any one of claims 1 to 8, wherein ultrasonic waves are transmitted into the body and reflected waves of the ultrasonic waves are received to acquire the measurement information in the acquisition step.

10. The method of any one of claims 1 to 9, further including a communication step, wherein
the activity score or a graph and/or a shape corresponding to the activity score is displayed on a screen of a computer terminal of a user via a wired or wireless communication line in the communication step.

11. A computer program product with a built-in program for automatic measurement of peristaltic movement, the program being able to implement the method of any one of claims 1 to 10 after being loaded and executed by a computer.

12. A device for automatic measurement of peristaltic movement, the device including: an acquirer that acquires measurement information about biological activity of one or more parts of a gastrointestinal tract; and
a calculator that extracts information about activity of the peristaltic movement from the information about the biological activity acquired by the acquirer, and obtains an activity score representing a degree of the activity of the peristaltic movement based on the information about the activity of the peristaltic movement.

13. A system for automatic measurement of peristaltic movement implemented via a wired or wireless communication line, the system including:
an acquisition device that acquires measurement information about biological activity of one or more parts of a gastrointestinal tract; and
a calculation device that extracts information about activity of the peristaltic movement from the information about the biological activity acquired by the acquisition device, and obtains an activity score representing a degree of the activity of the peristaltic movement based on the information about the activity of the peristaltic movement.
